# EUROPÄISCHE PATENTANMELDUNG

(11) **EP 1 475 112 A1**
(43) Veröffentlichungstag der Anmeldung: **10.11.2004**
(21) Anmeldenummer: 04008033.5
(22) Anmeldetag: 02.04.2004
(51) Int. Cl.: A61M 5/148

(54) **Vorrichtung zum Fördern von empfindlichen, flüssigen Medien**

(30) Priorität: 03.05.2003 DE 10319874
(71) Anmelder: Arzneimittel GmbH Apotheker Vetter & Co. Ravensburg, 88212 Ravensburg (DE)
(72) Erfinder: Böbst, Benjamin, 88441 Mittelbiberach (DE)
(74) Vertreter: Gleiss, Alf-Olav, Dipl.-Ing.

(57) **Zusammenfassung**

Es wird eine Vorrichtung zum Fördern von empfindlichen, flüssigen Medien, wie Lösungen, Dispersionen, Suspensionen und dergleichen vorgeschlagen, die mindestens einen einen Auslass aufweisenden, das Medium einschließenden flexiblen Behälter umfasst. Die Vorrichtung ist gekennzeichnet durch einen den Behälter (5) aufnehmenden Druckraum (3), in den der Behälter (5) unterbringbar und aus dem dessen Auslass (9) herausführbar ist, und durch eine mit dem Druckraum (3) verbindbare Druckquelle, mittels derer der Druckraum (3) mit Druck beaufschlagbar ist.

## Beschreibung

Die Erfindung betrifft eine Vorrichtung zum Fördern von empfindlichen, flüssigen Medien gemäß Oberbegriff des Anspruchs 1.

Im Bereich der Medizin ist es immer wieder erforderlich, empfindliche, flüssige Medien wie Lösungen, Dispersionen, Suspensionen und dergleichen zu fördern, beispielsweise Eiweißlösungen oder Ähnliches. Es ist bekannt, die Medien in flexible Behälter einzubringen und diese beispielsweise in einer bestimmten Höhe aufzuhängen, um das Medium unter einem von der Höhe der Aufhängung abhängigen Überdruck einer Verwendung zu zuführen, beispielsweise einer Filtrationseinrichtung. Bekannt ist es auch, ein gasförmiges Medium in den Behälter mit dem flüssigen Medium einzuleiten, um in diesem einen Überdruck aufzubauen und das flüssige Medium auszutreiben. Schließlich ist es bekannt, zum Fördern derartiger empfindlicher flüssiger Medien Schlauchpumpen einzusetzen.

Es hat sich herausgestellt, dass bei der Förderung der flüssigen Medien Nachteile auftreten. Wird der Behälter mit dem Medium in einer gewissen Höhe aufgehängt, so ist der Förderdruck vom Höhenunterschied zwischen dem Behälter und einem Verbraucher des Mediums abhängig. Wird der Behälter selbst mit einem Gas aufgeblasen, um einen Überdruck zum Fördern des flüssigen Mediums aufzubauen, so kann es zu Undichtigkeiten des Behälters und zum Austreten des flüssigen Mediums kommen. Selbst ein Platzen des Behälters ist nicht auszuschließen. Werden zum Fördern des flüssigen Mediums Schlauchpumpen eingesetzt, so werden einerseits empfindliche Bestandteile des flüssigen Mediums durch die Schlauchpumpe mechanisch belastet. Überdies ist es, insbesondere bei einem geringen Überdruck zum Fördern des flüssigen Mediums, häufig problematisch, einen definierten Überdruck einzustellen. Schließlich treten Pulsationen im geförderten Medium auf, was häufig unerwünscht ist.

Aufgabe der Erfindung ist es daher, eine Vorrichtung zum Fördern von empfindlichen flüssigen Medien bereitzustellen, die diese Nachteile nicht aufweist.

Zur Lösung dieser Aufgabe wird eine Vorrichtung vorgeschlagen, die die in Anspruch 1 genannten Merkmale aufweist. Sie zeichnet sich dadurch aus, dass ein Druckraum vorgesehen ist, in den der mit dem flüssigen Medium gefüllte Behälter einbringbar ist. Mit dem Druckraum wird eine Druckquelle verbunden, die den Druckraum mit einem Überdruck beaufschlagt und damit den flexiblen Behälter zusammenpresst. Damit wird das Medium aus diesem ausgetrieben. Besonders vorteilhaft ist es, dass der Druck sehr genau einstellbar ist und gut überwacht werden kann.

Weitere Ausgestaltungen ergeben sich aus den übrigen Unteransprüchen.

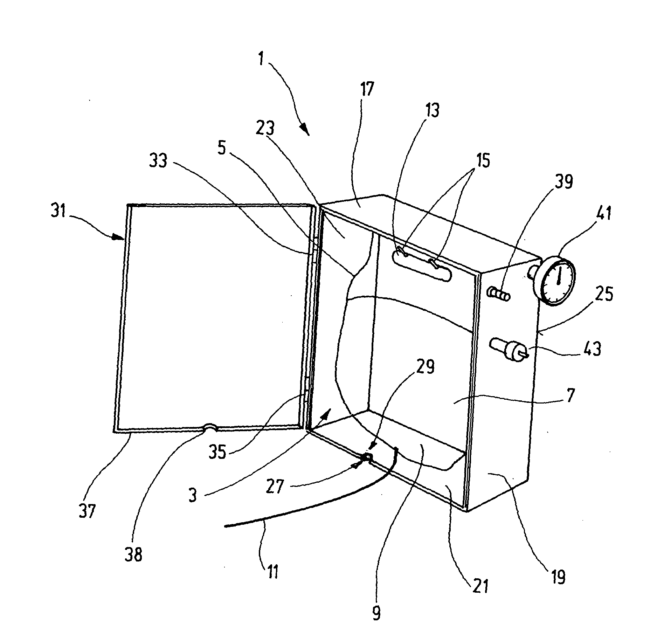

Die Erfindung wird im Folgenden anhand der Zeichnung näher erläutert. Diese zeigt einen als Druckschrank ausgebildeten Druckraum in perspektivischer Ansicht.

Der in der einzigen Figur gezeigte Druckschrank 1 weist einen als Druckraum 3 dienenden Innenraum auf, in den ein flexibler Behälter 5 eingebracht ist, in dem sich ein empfindliches flüssiges Medium 7 befindet.

Der Behälter 5 ist vorzugsweise als steriler Einwegbeutel ausgebildet, der also nur ein einziges Mal Verwendung findet, so dass die mit der Wiederbefüllung verbundenen Probleme der Reinigung und Desinfektion des Behälters entfallen.

Der Behälter 5 weist einen Auslass 9 auf, von dem ein Schlauch 11 ausgeht. Dieser dient zum Austragen des flüssigen Mediums 7 aus dem Behälter 5. Ansonsten ist der Behälter 5 nach außen vollkommen abgeschlossen, so dass das Medium 7 nicht kontaminiert werden kann.

Der Behälter 5 ist auf geeignete Weise im Inneren des Druckschranks 1 aufgehängt. Er weist hier eine übliche Ausnehmung 13 auf, die in einem doppelwandigen Bereich des Behälters 5 vorgesehen ist, so dass hier keine Verbindung zwischen dem flüssigen Medium 7 und der Außenwelt gegeben ist. In die Ausnehmung 13 greifen hier zwei Haken 15, die den Behälter 5 sicher im Inneren des Druckschranks 1 halten.

Eine derartige Aufhängung des Behälters 5 ist allerdings nur dann erforderlich, wenn der Druckschrank 1 im Stehen betrieben wird, was der Regelfall sein dürfte. Sollte dieser jedoch liegen, würde der Behälter 5 durch die Seitenwände 17, 19, 21 und 23, sicher gehalten, so dass es einer weiteren Befestigung des Behälters 5 im Druckraum 3 nicht bedürfte.

Die Haken 15 können an einer Rückwand 25 des Druckschranks 1 befestigt sein, oder aber von innen an der oberen Seitenwand 17. Entscheidend ist allein, dass der Behälter 5 hier in einer gewünschten Position gehalten wird, so dass er nicht aufgrund seines Eigengewichts den Schlauch 11 abknickt.

Die untere Seitenwand 21 weist eine Ausnehmung 27 auf, die vom Druckraum 3 nach außen führt und den Schlauch 11 aufnimmt. Es ist hier vorzugsweise eine elastische Schutzschicht, beispielsweise eine Gummitülle 29, vorgesehen, die ein Durchscheuern des Schlauchs 11 verhindert.

An einer Seitenwand 23 ist eine den Druckraum 3 druckdicht abschließende Tür 31 angebracht, beispielsweise über Scharniere 33 und 35 oder dergleichen.

Die Tür kann auf ihrer dem Druckraum 3 zugewandten Seite mit einer umlaufenden Dichtung 37 versehen werden, die mit den Stirnseiten der Seitenwände 17, 19, 21 und 23 so zusammenwirkt, dass der Druckraum 3 druckdicht abgeschlossen ist.

Unten an der Tür 31 kann wiederum eine Schutzschicht 38 vorgesehen werden, die den Schlauch 11 sicher in der Ausnehmung 27 hält, ohne diesen durchzuscheuern.

An einer der Seitewände, hier an der Seitenwand 19, ist ein Druckanschluss 39 vorgesehen, über den der Druckraum 3 mit einer hier nicht dargestellten Druckquelle verbunden werden kann. Diese kann den Druckraum 3 bei geschlossener Tür 31 unter einen Überdruck setzen, damit auch den flexiblen Behälter 5. An der Seitenwand 19 ist außerdem eine Druckanzeige 41 vorgesehen, schließlich noch ein Überdruckventil 43.

Die Vorrichtung 1 kann schließlich noch mit einer Rütteleinheit versehen werden, die dafür sorgt, dass das im Behälter 5 vorhandene Medium 7 kontinuierlich oder während vorgebbarer Zeiträume in Bewegung gehalten wird, um ein Absetzen der im Medium vorhandenen Substanzen zu vermeiden.

Die Rütteleinheit kann unmittelbar auf den Behälter 5 wirken und im Druckraum 3 untergebracht sein. Vorzugsweise wird jedoch der Druckschrank 1 auf einen Rütteltisch gestellt und damit in seiner Gesamtheit einer Bewegung unterworfen, die sich auf das Medium 7 im Behälter 5 auswirkt.

Im Folgenden wird auf die Funktion der Vorrichtung zum Fördern empfindlicher flüssiger Medien näher eingegangen:

In dem Behälter 5 befindet sich ein empfindliches Medium, beispielsweise eine Lösung, Dispersion oder Suspension, die keinen mechanischen Belastungen unterworfen werden sollte, wie sie beispielsweise bei Einsatz einer Schlauchpumpe gegeben sind.

Der Behälter 5 selbst ist flexibel, so dass ein auf den Behälter 5 wirkender Überdruck sich auf das flüssige Medium 7 in dessen Inneren auswirkt und dieses über den Auslass 9 und den Schlauch 11 austreibt.

Der Behälter 5 wird in das Innere des Druckschranks 1, also in den Druckraum 3 eingebracht und vorzugsweise an den Haken 15 befestigt. Der Schlauch 11 wird durch die Ausnehmung 27 geführt, deren Abmessung so gewählt ist, dass der Schlauch 11 beim Schließen der Tür 31 nicht abgequetscht wird. Der Schlauch 11 kann aus einem flexiblen Material bestehen, das die Ausnehmung 27 druckdicht abschließt. Vorzugsweise wird hier aber eine Schutzschicht 29 vorgesehen, die mit der Schutzschicht 38 an der Tür 31 so zusammenwirkt, dass beim Schließen der Tür der Schlauch 11 vollständig umschlossen ist und der Druckraum 3 druckdicht nach außen abgeschlossen wird. Zusätzlich wirkt hier die Dichtung 37 mit den Seitenwänden 17, 19, 21 und 23 zusammen, so dass beim Schließen der Tür 31 der Druckraum 3 vollständig abgeschlossen ist.

Nun wird durch den Druckanschluss 39 ein Überdruck im Inneren der Vorrichtung, hier im Druckschrank 1 aufgebaut. Vorzugsweise wird ein gasförmiges Medium in den Druckraum 3 eingeleitet, weil dieses beim Öffnen der Tür 31 schnell entweicht. Denkbar wäre es aber auch, ein flüssiges Medium einzuleiten, weil dieses vollständig durch den Behälter 5 von dem flüssigen Medium 7 im Inneren des Behälters getrennt wird.

Vorzugsweise wird Stickstoff in den Druckraum 3 geleitet, weil dieser dass empfindliche Medium 7 im Inneren des Behälters 5 auch bei irgendwelchen Undichtigkeiten des Behälters nicht beeinträchtigt. Außerdem wird die Innenseite des Druckschrankes 1 durch dieses Gas nicht kontaminiert.

Der im Druckraum 3 wirkende Überdruck kann durch eine Überwachungseinrichtung in der Höhe und in dem über der Zeit gegebenen Druckverlauf eingestellt werden. Dazu kann eine geeignete Steuerung der Druckquelle vorgesehen werden. Eine Überwachung des Druckraums 3 kann durch eine Überwachungseinrichtung realisiert werden, die einerseits die Druckanzeige 41 und andererseits das Überdruckventil 43 aufweist, welches einen gefährlichen Überdruck im Druckraum 3 verhindert und damit ein ungewolltes Aufsprengen der Tür 31. Besonders wichtig ist jedoch, dass das Medium 7 mit nur einem definierten, für das Medium 7 und für dessen Verarbeitung ungefährlichen Druck aus dem Behälter 5 ausgetragen wird.

Vorzugsweise wird ein auf einen gewünschten Druck einstellbares Überdruckventil 43 eingesetzt, um die Vorrichtung ohne weiteres für verschiedene Förderzwecke einzusetzen. Beispielsweise beim Filtrieren empfindlicher Medien wird ein Filtrationsdruck von etwa 0,2 bar eingesetzt. Entsprechend muss dann das Überdruckventil 43 auf diesen Druck eingestellt werden, um die Verwendbarkeit der Vorrichtung zum Filtrieren zu ermöglichen.

Grundsätzlich ist es also möglich, mit einer entsprechenden Überwachungseinrichtung und mit einstellbaren Druckquellen einen gewünschten Druck im Druckraum 3 aufrechtzuerhalten oder aber auch unterschiedliche Druckwerte zu verschiedenen Zeiten. Damit ist die Vorrichtung universell einsetzbar, also nicht nur zum Filtrieren von Medien, sondern für verschiedene Verarbeitungs- und Darreichungszwecke, gegebenenfalls auch als Dosiereinrichtung beim Verabreichen von Medikamenten.

Bei dem in der Figur dargestellten Ausführungsbeispiel ist der Druckraum 3 so ausgelegt, dass ein einziger Behälter 5 untergebracht werden kann. Denkbar ist es aber auch, den Druckraum unterteilbar zu machen und den verschiedenen druckdicht voneinander abgegrenzten Bereichen des Druckraums unterschiedliche Druckwerte zuzuordnen und in diesen Bereichen auch unterschiedliche Behälter unterzubringen.

Entscheidend ist, dass das Medium 7 aus dem Behälter 5 berührungslos gefördert und ausgebracht werden kann: Durch einen von außen auf den flexiblen Behälter 5 wirkenden Druck wird das Medium 7 über den Auslass 9 und den Schlauch 11 aus dem Behälter 5 gefördert und verschiedenen Verwendungszwecken zugeführt.

Der Druckraum 3 wird mit dem Medium 7 nicht in Berührung gebracht, so dass ein Austausch von Behältern mit unterschiedlichen Medien sehr leicht möglich ist, ohne dass es aufwendiger Desinfektionsverfahren bedürfte. Die Medien unterschiedlicher Behälter kommen weder mit dem Innenraum beziehungsweise Druckraum des Druckschranks in Berührung noch mit irgendwelchen mit dem Druckraum verbundenen Geräten, hier dem Druckanschluss 39, der Druckanzeige 41 oder dem Überdruckventil 43. Wenn vorzugsweise der Druckraum 3 mit unter Überdruck stehendem Stickstoff beaufschlagt wird, kann sichergestellt werden, dass das Medium 7 nicht beeinträchtigt wird, selbst wenn der Behälter 5 eine undichte Stelle haben sollte und das Druckmedium in das Innere des Behälters 5 eindringt.

Es zeigt sich nach allem auch, dass die Vorrichtung sehr einfach aufgebaut ist und damit praktisch störungsfrei arbeiten kann. Dies führt zu einem sehr sicheren Einsatz der hier beschriebenen Vorrichtung, was gerade im medizinischen Bereich von entscheidender Bedeutung ist.

Um die Vorrichtung auch bei sehr geringen Überdruckwerten einsetzen zu können, kann der Druckanschluss 39 auch mit einem Druckreduzierventil versehen werden. Dies führt zu einer zusätzlichen Sicherheit, wenn die Vorrichtung beispielsweise bei einer Filtration eingesetzt wird, bei der ein Überdruck von nur 0,2 bar gewünscht ist.

Die Anforderungen an den Behälter 5 sind in keiner Weise speziell. Es können die üblichen sterilen Einwegbeutel eingesetzt werden, wie sie auf dem Markt vorhanden sind. Es bedarf also keinerlei Anpassung des Behälters bei der Verwendung in der hier beschriebenen Vorrichtung.

Auch für den Fall, dass das Medium ständig in Bewegung gehalten werden muss, bedarf es hier keiner besonderen Vorkehrungen: Der Druckschrank 1 kann auf übliche Rütteltische aufgebracht werden, die dann das Medium 7 mit der Rüttelbewegung beaufschlagen. Da der Druckschrank 1 sehr einfach und robust aufgebaut ist, sind für diesen keinerlei Nachteile bei Einsatz eines Rütteltisches zu erwarten.

## Patentansprüche

1. Vorrichtung zum Fördern von empfindlichen flüssigen Medien, wie Lösungen, Dispersionen, Suspensionen und dergleichen mit mindestens einem einen Auslass aufweisenden das Medium einschließenden flexiblen Behälter, **gekennzeichnet durch** einen den Behälter (5) aufnehmenden Druckraum (3), in den der Behälter (5) unterbringbar und aus dem dessen Auslass (9) herausführbar ist, und **durch** eine mit dem Druckraum (3) verbindbare Druckquelle, mittels derer der Druckraum (3) mit Druck beaufschlagbar ist.

2. Vorrichtung nach Anspruch 1, **dadurch gekennzeichnet, dass** die Druckquelle den Druckraum (3) mit Stickstoff beaufschlagt.

3. Vorrichtung nach Anspruch 1 oder 2, **gekennzeichnet durch** eine Überwachungseinrichtung, mittels derer ein definierter Druck und/oder ein definierter Druckverlauf über der Zeit einstellbar ist.

4. Vorrichtung nach einem der vorhergehenden Ansprüche, **dadurch gekennzeichnet, dass** der Behälter (5) als Beutel, vorzugsweise als steriler Einwegbeutel ausgelegt ist.

5. Vorrichtung nach einem der vorhergehenden Ansprüche, **gekennzeichnet durch** eine Rütteleinheit, die auf das aus dem Behälter (5) auszutragende Medium (7) wirkt.

6. Vorrichtung nach einem der vorhergehenden Ansprüche, **dadurch gekennzeichnet, dass** die Rütteleinheit einen Rütteltisch aufweist, auf dem der Druckraum (3) anordenbar ist.
